(19) Europäisches Patentamt
European Patent Office
Office européen des brevets

(11) Veröffentlichungsnummer: **0 004 644**
**A2**

# (12) EUROPÄISCHE PATENTANMELDUNG

(21) Anmeldenummer: **79100946.7**

(22) Anmeldetag: **29.03.79**

(51) Int. Cl.²: **C 07 F 9/65**
A 01 N 9/36
//C07D239/46, C07D239/56

(30) Priorität: **07.04.78 DE 2814984**

(43) Veröffentlichungstag der Anmeldung:
**17.10.79 Patentblatt 79/21**

(84) Benannte Vertragsstaaten:
**BE CH DE FR GB IT NL**

(71) Anmelder: **BAYER Aktiengesellschaft**
**Zentralbereich Patente,Marken und Lizenzen Bayerwerk**
**D-5090 Leverkusen 1(DE)**

(72) Erfinder: **Maurer, Fritz, Dr.**
**Roeberstrasse 8**
**D-5600 Wuppertal 1(DE)**

(72) Erfinder: **Hammann, Ingeborg, Dr.**
**Belfortstrasse 9**
**D-5000 Köln(DE)**

(72) Erfinder: **Homeyer, Bernhard, Dr.**
**Obere Strasse 28**
**D-5090 Leverkusen 3(DE)**

(54) **Pyrimidin(5)yl-(thiono)(thiol)-phosphor(phosphon)-säureester bzw. -esteramide, Verfahren zu ihrer Herstellung und ihre Verwendung als Insektizide und Akarizide.**

(57) Die Erfindung betrifft neue Pyrimidin(5)yl-(thiono)-(thiol)-phosphor(phosphon)-säureester bzw. -esteramide der Formel

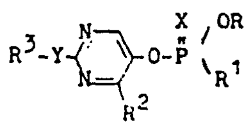

in welcher
R für Alkyl,
$R^1$ für Alkyl, Aryl, Alkoxy, Alkylthio oder Alkylamino,
$R^2$ für Wasserstoff oder Alkyl,
$R^3$ für Alkyl,
X für Sauerstoff oder Schwefel und
Y für Sauerstoff, Schwefel oder Alkylamino steht.

Die neuen Verbindungen zeichnen sich durch starke insektizide und akarizide Wirksamkeit aus.

EP 0 004 644 A2

Croydon Printing Company Ltd.

0004644



BAYER AKTIENGESELLSCHAFT
Zentralbereich
Patente, Marken und Lizenzen

5090 Leverkusen, Bayerwerk
Ad-Ma / Typ Ia

# Pyrimidin(5)yl-(thiono)(thiol)-phosphor(phosphon)-säureester bzw. -esteramide, Verfahren zu ihrer Herstellung und ihre Verwendung als Insektizide und Akarizide

Die Erfindung betrifft neue Pyrimidin(5)yl-(thiono)-(thiol)-phosphor(phosphon)-säureester bzw. -esteramide, welche insektizide und akarizide Eigenschaften besitzen, sowie ein Verfahren zu ihrer Herstellung.

Es ist bekannt, daß 2-Alkoxy- und 2-Alkylthiopyrimidin-(4)yl-thionophosphorsäureester, wie z.B. O,O-Diäthyl-O-(2-methoxy-6-methyl-pyrimidin(4)yl)- und O,O-Diäthyl-O-(2-methylthio-6-methyl-pyrimidin(4)yl)-thionophosphorsäureester, insektizide und akarizide Eigenschaften aufweisen (vergleiche DT-AS 91o 652 und DT-OS 2 343 931).

Es wurden die neuen Pyrimidin(5)yl-(thiono)(thiol)-phosphor(phosphon)-säureester bzw. -esteramide der Formel

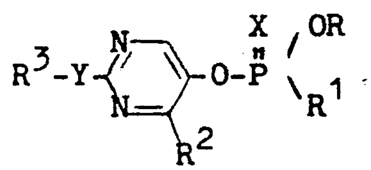 (I)

in welcher

R für Alkyl,

$R^1$ für Alkyl, Aryl, Alkoxy, Alkylthio oder Alkylamino,

$R^2$ für Wasserstoff oder Alkyl,

$R^3$ für Alkyl,

X für Sauerstoff oder Schwefel und

Y für Sauerstoff, Schwefel oder Alkylimino steht,

synthetisiert.

Die neuen Verbindungen zeichnen sich durch starke insektizide und akarizide Wirksamkeit aus.

Weiter wurde gefunden, daß man die neuen Pyrimidin(5)yl-(thiono)(thiol)-phosphor(phosphon)-säureester bzw. -esteramide der Formel I erhält, wenn man (Thiono)(thiol)-phosphor(phosphon)-säureesterhalogenide bzw. (Thiono)-phosphorsäureesteramidhalogenide der Formel

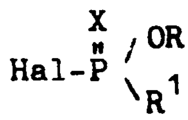

(II)

worin R, $R^1$ und X die oben angegebene Bedeutung haben und Hal für Chlor oder Brom steht,

mit 5-Hydroxy-pyrimidinen der Formel

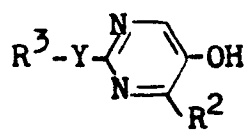

(III)

worin $R^2$, $R^3$ und Y die oben angegebene Bedeutung haben,

gegebenenfalls in Gegenwart eines Säureakzeptors und gegebenenfalls in Gegenwart eines inerten Verdünnungsmittels umsetzt.

Überraschenderweise zeigen die erfindungsgemäßen Pyrimidin(5)yl-(thiono)(thiol)-phosphor(phosphon)-säureester bzw. -esteramide eine wesentlich bessere insektizide und akarizide Wirkung als die entsprechenden aus dem Stand der Technik vorbekannten Verbindungen analoger Konstitution und gleicher Wirkungsrichtung.

Die Produkte gemäß vorliegender Erfindung stellen somit eine echte Bereicherung der Technik dar.

Verwendet man 2-Methoxy-5-hydroxy-pyrimidin und O,O-Diäthyl-thionophosphorsäurediesterchlorid als Ausgangsstoffe, so kann der Reaktionsablauf durch folgendes Formelschema wiedergegeben werden:

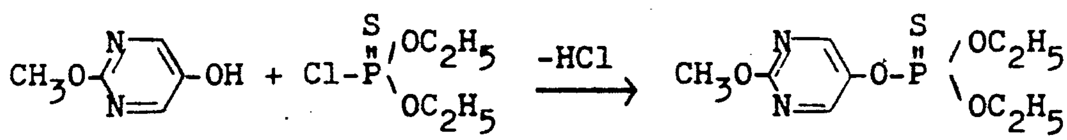


Die als Ausgangsstoffe zu verwendenden (Thiono)(thiol)-phosphor(phosphon)-säureesterhalogenide bzw. (Thiono)-phosphorsäureesteramidhalogenide sind durch Formel II allgemein definiert.

Vorzugsweise stehen darin jedoch

R für geradkettiges oder verzweigtes Alkyl mit 1 bis 5, insbesondere 1 bis 3 Kohlenstoffatomen,

$R^1$ für geradkettiges oder verzweigtes Alkyl mit 1 bis 5, insbesondere 1 bis 3 Kohlenstoffatomen, Phenyl, geradkettiges oder verzweigtes Alkoxy, Alkylthio oder Alkylamino mit jeweils 1 bis 5, insbesondere 1 bis 3 Kohlenstoffatomen je Alkylrest,

X für Sauerstoff oder Schwefel und

Hal für Chlor.

Die erfindungsgemäß zu verwendenden (Thiono)(thiol)-phosphor(phosphon)-säureesterhalogenide sind bereits bekannt. Als Beispiele seien genannt:

O-Methyl-, O-Äthyl-, O-n-Propyl-, O-iso-Propyl-, -methan-, -äthan-, -propan- bzw. -phenylphosphonsäureesterhalogenide und die entsprechenden Thionoanalogen, ferner O,O-Dimethyl-, O,O-Diäthyl-, O,O,-Di-n-propyl-, O-Methyl-O-äthyl-, O-Methyl-O-n-propyl-, O-Methyl-O-iso-propyl-, O-Äthyl-O-n-propyl- und O-Äthyl-O-iso-propyl-phosphorsäurediesterchlorid und die entsprechenden Thionoanalogen, ferner O,S-Dimethyl-, O,S-Diäthyl-, O,S-Di-n-propyl-, O,S-Di-iso-propyl, O-Äthyl-Sn-propyl-, O-Äthyl-S-iso-propyl-, O-Äthyl-S-sec.-butyl-, O-n-Propyl-S-äthyl-, O-n-Propyl-S-iso-propyl- und O-iso-Propyl-S-n-propylthiolphosphorsäurediesterchlorid und die entsprechenden Thionoanalogen, ferner O-Methyl-N-methyl-, O-Methyl-N-äthyl-, O-Methyl-N-propyl-, O-Methyl-N-iso-propyl-, O-Äthyl-N-methyl-, O-Äthyl-N-äthyl-, O-Äthyl-N-n-propyl-, O-Äthyl-N-iso-propyl-, O-n-Propyl-N-methyl-, O-n-Propyl-N-äthyl-, O-n-Propyl-N-n-propyl-, O-n-Propyl-N-iso-propyl-, O-iso-Propyl-N-methyl-, O-iso-Propyl-N-äthyl-, O-iso-Propyl-N-n-propyl- und O-iso-Propyl-N-iso-propyl-phosphorsäureesteramidchlorid und die entsprechenden Thionoanalogen.

Die als weitere Ausgangsverbindungen zu verwendenden 5-Hydroxy-pyrimidine sind durch Formel III allgemein definiert.

Vorzugsweise stehen darin

$R^2$ für Wasserstoff oder Methyl,

$R^3$ für geradkettiges oder verzweigtes Alkyl mit 1 bis 5, insbesondere 1 bis 3 Kohlenstoffatomen und

Y für Sauerstoff, Schwefel oder Alkylimino, wobei letzteres 1 bis 5, insbesondere 1 bis 3 Kohlenstoffatome je Alkylrest enthält.

Die erfindungsgemäß verwendbaren 5-Hydroxy-pyrimidine sind zum Teil in der Literatur beschrieben. So erhält man z.B. die 2-Alkylthio-5-hydroxypyrimidine durch Umsetzung von 2-Alkylthio-5-methoxypyrimidinen mit wäßriger Ammoniaklösung im Autoklaven bei 18o-19o°C (vergleiche Collect. Czech. Chem. Commun. 4o (1975), S. 1o78-1o88). Die 2-Dialkylamino-5-hydroxy-pyrimidine werden durch Erwärmen von 2-Dialkylamino-5-alkoxy- insbesondere -5-methoxy-pyrimidinen mit wäßrig-alkoholischen Alkalilaugen auf 19o°C im Autoklaven und anschließendes schwaches Ansäuern hergestellt. In analoger Weise erhält man die 2-Alkoxyverbindungen. Die 2-Dialkylamino-5-alkoxy-pyrimidine entstehen, wenn man 1,1,2-Trialkoxyäthane mit einem Gemisch aus Phosgen und Dimethylformamid nach Art einer Vilsmeyer-Reaktion umsetzt und das anfallende Reaktionsgemisch sogleich mit einer Lösung eines Dialkylguanidinsalzes und Alkalialkoholat in einem Alkohol umsetzt (vergleiche DT-AS 1 145 622).

Als Beispiele für die erfindungsgemäß verwendbaren 5-Hydroxy-pyrimidine seien im einzelnen genannt:
2-Methoxy-, 2-Äthoxy-, 2-n-Propoxy- und 2-iso-Propoxy-5-hydroxy-pyrimidin,
2-Methylthio-, 2-Äthylthio-, 2-n-Propylthio- und 2-iso-Propylthio-5-hydroxy-pyrimidin,
2-Dimethylamino-, 2-Diäthylamino-, 2-Dipropylamino- und 2-Di-iso-propylamino-5-hydroxy-pyrimidin,
2-Methoxy-, 2-Äthoxy-, 2-n-Propoxy- und 2-iso-Propoxy-4-methyl-5-hydroxy-pyrimidin, 2-Methylthio-, 2-Äthylthio-, 2-n-Propylthio- und 2-iso-Propylthio-4-methyl-5-hydroxy-pyrimidin, 2-Dimethylamino-, 2-Diäthylamino, 2-Di-n-propylamino- und 2-Di-iso-propylamino-4-methyl-5-hydroxy-pyrimidin.

Das Verfahren zur Herstellung der neuen Pyrimidin(5)yl-(thiono)(thiol)-phosphor(phosphon)-säureester bzw. -esteramide wird bevorzugt unter Verwendung geeigneter Lösungs- oder Verdünnungsmittel durchgeführt. Als solche kommen praktisch alle inerten organischen Solventien infrage. Hierzu gehören insbesondere aliphatische und aromatische, gegebenenfalls chlorierte, Kohlenwasserstoffe, wie Benzin, Benzol, Toluol, Xylol, Methylenchlorid, Chloroform, Tetrachlorkohlenstoff, Chlorbenzol und o-Dichlorbenzol, Äther wie Diäthyl- und Dibutyläther, Tetrahydrofuran und Dioxan, Ketone wie Aceton, Methyläthyl-, Methylisopropyl- und Methylisobutylketon sowie Nitrile wie Acetonitril und Propionitril.

Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -äthylat, ferner aliphatische, aromatische oder heterocyclische Amine, beispielsweise Triäthylamin,Trimethylamin, Dimethylanilin, Dimethylbenzylamin und Pyridin.

Die Reaktionstemperatur kann innerhalb eines größeren Bereichs variiert werden. Im allgemeinen arbeitet man zwischen 0 und 8o°C, vorzugsweise bei 2o bis 6o°C.

Das erfindungsgemäße Verfahren wird im allgemeinen bei Normaldruck durchgeführt.

Zur Durchführung des erfindungsgemäßen Verfahrens werden die Ausgangsstoffe gewöhnlich in äquimolaren Mengen eingesetzt. Ein Überschuß der einen oder anderen Reaktionskomponente bringt keine wesentlichen Vorteile. Die Umsetzung wird im allgemeinen in einem geeigneten Verdünnungsmittel in Gegenwart eines Säureakzeptors durchgeführt und das Reaktionsgemisch wird mehrere Stunden bei der erforderlichen Temperatur gerührt. Danach gibt man ein organisches Lösungsmittel, z.B. Toluol, zu und arbeitet die organische Phase wie üblich durch Waschen, Trocknen und Abdestillieren des Lösungsmittels auf.

Die neuen Verbindungen fallen zum Teil in Form von Ölen an, die sich zum Teil nicht unzersetzt destillieren lassen, jedoch durch sogenanntes "Andestillieren", d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Soweit die neuen Produkte nach dem Abdestillieren des Lösungsmittels in fester Form anfallen, werden sie durch Umkristallisation gereinigt und durch ihren Schmelzpunkt charakterisiert.

0004644

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hyginesektor vorkommen. Sie sind gegen normal sensible und resistente Arten sowie gegen alle oder einezlne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophaus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips fermoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuders sowie durch parenterale Anwendung in Form beispielsweise der Injektion.

0004644

Beispiel A

Phaedon-Larven-Test

Lösungsmittel: 3 Gewichtsteile
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 1, 3, 4, 5, 6, 12, 13 und 14.

0004644

Beispiel B

Tetranychus-Test (resistent)

Lösungsmittel: 3 Gewichtsteile
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 1, 3, 4, 6, 7, 9, 12, 13 und 14.

Beispiel C

Grenzkonzentrations-Test / Bodeninsekten

| | | | |
|---|---|---|---|
| Testinsekt: | | | (im Boden) |
| Lösungsmittel: | 3 | Gewichtsteile | Aceton |
| Emulgator: | 1 | Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden,welche in ppm ( = mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 2, 3, 4, 5, 6, 10, 12, 13 und 14.

Beispiel D

Grenzkonzentrations-Test / Wurzelsystemische Wirkung

Testinsekt:
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 2, 3, 4, 8, 9, 10, 12 und 14.

Herstellungsbeispiele

Beispiel 1: 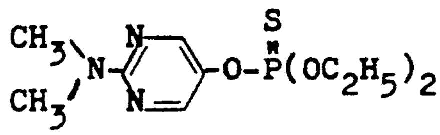

Ein Gemisch aus 3oo ml Acetonitril, 13,9 g (o,1 Mol) 2-Dimethylamino-5-hydroxy-pyrimidin, 2o,7 g (o,15 Mol) Kaliumcarbonat und 18,8 g (o,1 Mol) O,O-Diäthylthionophosphorsäurediesterchlorid wird 2 Stunden bei 45°C gerührt. Dann gießt man das Reaktionsgemisch in 4oo ml Toluol und wäscht es zweimal mit je 3oo ml Wasser. Die Toluollösung wird über Natriumsulfat getrocknet und im Vakuum eingedampft. Den Rückstand destilliert man im Hochvakuum an. Man erhält so 23 g (79 % der Theorie) O,O-Diäthyl-O-(2-dimethylamino-pyrimidin(5)yl)-thionophosphorsäureester in Form eines braunen Öles mit dem Brechungsindex $n_D^{21}$: 1,529o.

In analoger Weise können die folgenden Verbindungen der Formel

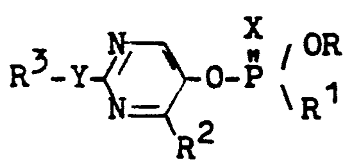 (I)

hergestellt werden:

| Beispiel Nr. | R | $R^1$ | $R^2$ | $R^3$ | Y | X | Ausbeute (% der Theorie) | Physikal. Daten (Brechungsindex; Schmelzpunkt °C) |
|---|---|---|---|---|---|---|---|---|
| 2 | $C_2H_5$ | $OC_2H_5$ | H | $CH_3$ | S | S | 63 | $n_D^{21}$:1,5388 |
| 3 | $CH_3$ | $OCH_3$ | H | $CH_3$ | $N-CH_3$ | S | 76 | 54 |
| 4 | $C_2H_5$ | $CH_3$ | H | $CH_3$ | $N-CH_3$ | S | 8o | $n_D^{21}$:1,55oo |
| 5 | $C_2H_5$ | 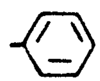 | H | $CH_3$ | $N-CH_3$ | S | 67 | 66 |
| 6 | $C_2H_5$ | $OC_2H_5$ | H | $C_2H_5$ | $N-C_2H_5$ | S | 8o | $n_D^{22}$:1,5113 |
| 7 | $C_2H_5$ | $SC_3H_7$-n | H | $CH_3$ | $N-CH_3$ | S | 63 | $n_D^{22}$:1,56o4 |
| 8 | $C_2H_5$ | $NH-C_3H_7$-iso | H | $CH_3$ | $N-CH_3$ | 0 | 79 | 78 |
| 9 | $C_2H_5$ | $NH-C_3H_7$-iso | H | $C_2H_5$ | $N-C_2H_5$ | 0 | 46 | $n_D^{22}$:1,51o7 |
| 1o | $C_2H_5$ | $NH-C_3H_7$-iso | H | $CH_3$ | $N-CH_3$ | S | 38 | 8o |
| 11 | $C_2H_5$ | $OC_2H_5$ | H | $CH_3$ | $N-CH_3$ | 0 | | |

| Bei-spiel Nr. | R | $R^1$ | $R^2$ | $R^3$ | Y | X | Ausbeute (% der Theorie) | Physikal. Daten (Brechungsindex; Schmelzpunkt °C) |
|---|---|---|---|---|---|---|---|---|
| 12 | $C_3H_7$-n | $OC_2H_5$ | H | $CH_3$ | N-$CH_3$ | S | 77 | $n_D^{22}$:1,5232 |
| 13 | $CH_3$ | $OC_3H_7$-n | H | $CH_3$ | N-$CH_3$ | S | 83 | $n_D^{22}$:1,53o3 |
| 14 | $CH_3$ | $OC_2H_5$ | H | $CH_3$ | N-$CH_3$ | S | 83 | $n_D^{22}$:1,5357 |
| 15 | $C_2H_5$ | $OC_2H_5$ | H | $CH_3$ | O | S | | |
| 16 | $C_2H_5$ | $OC_2H_5$ | H | $C_3H_7$-iso | O | S | | |
| 17 | $C_2H_5$ | $OC_2H_5$ | H | $C_3H_7$-n | S | S | | |
| 18 | $C_2H_5$ | $OC_2H_5$ | $CH_3$ | $CH_3$ | N-$CH_3$ | S | | |
| 19 | $C_2H_5$ | $OC_2H_5$ | H | $C_3H_7$-n | N-$C_3H_7$-n | S | | |
| 2o | $CH_3$ | $OCH_3$ | H | $C_2H_5$ | N-$C_2H_5$ | S | | |

| Beispiel-Nr. | R | $R^1$ | $R^2$ | $R^3$ | Y | X | Ausbeute (% der Theorie) | Physikal. Daten (Brechungsindex; Schmelzpunkt °C) |
|---|---|---|---|---|---|---|---|---|
| 21 | $CH_3$ | $CH_3$ | H | $CH_3$ | $N-CH_3$ | S | | |
| 22 | $CH_3$ | $NH-C_3H_7$-iso | H | $CH_3$ | $N-CH_3$ | S | | |
| 23 | $C_2H_5$ | $C_2H_5$ | H | $CH_3$ | $N-CH_3$ | S | | |
| 24 | $C_2H_5$ | $S-C_3H_7$-n | H | $CH_3$ | $N-CH_3$ | O | | |
| 25 | $CH_3$ | $OC_2H_5$ | H | $C_2H_5$ | $N-C_2H_5$ | S | | |
| 26 | $C_3H_7$-n | $OCH_3$ | H | $C_2H_5$ | $N-C_2H_5$ | S | | |

Die als Ausgangsmaterialien einzusetzenden 5-Hydroxy-pyrimidine können z.B. wie folgt hergestellt werden:

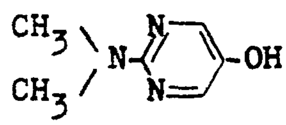

Eine Lösung von 69 g (o,45 Mol) 2-Dimethylamino-5-methoxy-pyrimidin (Herstellung s. unten) und 38 g (o,68 Mol) Kaliumhydroxid in einem Gemisch aus 8o ml Wasser und 17o ml Methanol wird in einem Autoklaven 4 Stunden auf 19o°C erhitzt. Dann destilliert man das Methanol im Vakuum ab, versetzt den Rückstand mit 5o ml Eiswasser und bringt die Lösung unter Kühlen durch Zugabe von konzentrierter Salzsäure auf einen pH-Wert von 4,5. Nach 3o Minuten saugt man das auskristallisierte Produkt ab und erhält so 34,4 g (55 % der Theorie) 2-Dimethylamino-5-hydroxy-pyrimidin als schwach braun gefärbtes Kristallpulver mit dem Schmelzpunkt 163°C.

Analog können die folgenden Verbindungen der Formel

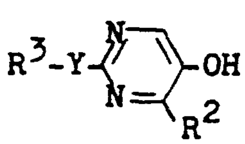

hergestellt werden:

| $R^2$ | $R^3$ | Y | Ausbeute (% der Theorie) | Schmelzpunkt °C |
|---|---|---|---|---|
| H | $CH_3$ | S | 33 | 154 |
| H | $C_2H_5$ | $N-C_2H_5$ | 58 | 8o |
| H | $CH_3$ | O | | |
| H | $C_3H_7$-iso | O | | |
| H | $C_3H_7$-n | S | | |
| $CH_3$ | $CH_3$ | $N-CH_3$ | | |
| $CH_3$ | $CH_3$ | S | | |
| $CH_3$ | $CH_3$ | $N-C_2H_5$ | | |
| H | $C_3H_7$-n | $N-C_3H_7$-n | | |

Herstellung von 2-Dimethylamino-5-methoxypyrimidin:

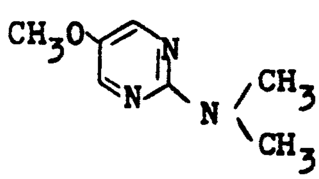

In eine Lösung von 146 g (2 Mol) Dimethylformamid in 6oo ml Methylenchlorid leitet man bei 0-1o°C 2oo g

(~2 Mol) Phosgen. Anschließend gibt man 12o g (1 Mol) 1,1,2-Trimethoxyäthan zu und kocht das Reaktionsgemisch 16 Stunden unter Rückfluß. Nach Abkühlen auf 0-5°C wird eine Lösung von Natriummethylat in Methanol zugegeben, bis ein pH-Wert von 8 erreicht ist (ca. 2 Mol Natriummethylat sind dazu erforderlich). Man saugt vom ausgefallenen Salz ab, wäscht mit Methylenchlorid nach und dampft das Filtrat im Vakuum ein. Der ölige Rückstand wird zu einer Mischung aus 1,1 Mol Natriummethylat, 5oo ml Methanol und 136 g (1 Mol) Dimethylguanidin-Sulfat gegeben und 18 Stunden unter Rückfluß gekocht. Man saugt vom ausgefallenen Salz ab, und dampft das Filtrat im Vakuum ein. Der Rückstand wird in 8oo ml Methylenchlorid gelöst, 1 mal mit 2oo ml Wasser gewaschen, über Natriumsulfat getrocknet und im Vakuum eingedampft. Dann destilliert man das Produkt. Man erhält auf diese Weise 94 g (62% der Theorie) 2-Dimethylamino-5-methoxy-pyrimidin in Form einer farblosen Flüssigkeit mit dem Siedepunkt 7o°C/o,2 mm Hg.

In analoger Weise können die folgenden Verbindungen hergestellt werden:

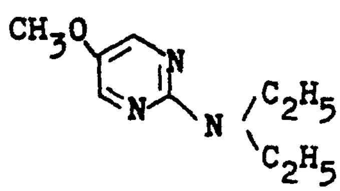

in 56%iger Ausbeute der Theorie mit einem Siedepunkt von 78°C/o,2 mm Hg

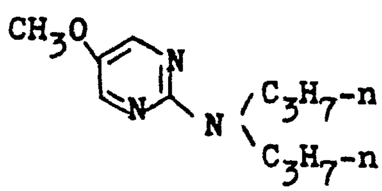

0004644

Patentansprüche:

1. Pyrimidin(5)yl-(thiono)(thiol)-phosphor(phosphon)-säureester bzw. -esteramide der Formel

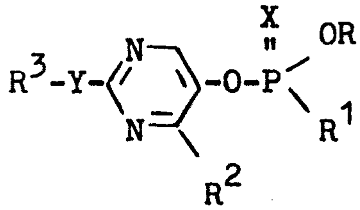

(I)

in welcher
R für Alkyl,
$R^1$ für Alkyl, Aryl, Alkoxy, Alkylthio oder Alkylamino,
$R^2$ für Wasserstoff oder Alkyl,
$R^3$ für Alkyl,
X für Sauerstoff oder Schwefel und
Y für Sauerstoff, Schwefel oder Alkylimino steht.

2. Verfahren zur Herstellung der Pyrimidin(5)yl-(thiono)(thiol)-phosphor(phosphon)-säureester bzw. -esteramide der Formel I, dadurch gekennzeichnet, daß man (Thiono)(thiol)-phosphor(phosphon)-säureesterhalogenide bzw. (Thiono)-phosphorsäureesteramidhalogenide der Formel

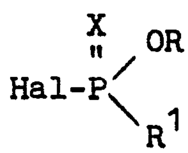

(II)

worin
R, $R^1$ und X die im Anspruch 1 angegebene Bedeutung haben und
Hal für Chlor oder Brom steht,

mit 5-Hydroxy-pyrimidinen der Formel

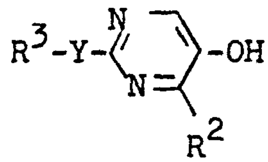

(III)

worin

$R^2$, $R^3$ und Y die in Anspruch 1 angegebene Bedeutung haben,

gegebenenfalls in Gegenwart eines Säureakzeptors und gegebenenfalls in Gegenwart eines inerten Verdünnungsmittels umsetzt.

3. Insektizide und akarizide Mittel, gekennzeichnet durch einen Gehalt an Verbindungen gemäß Anspruch 1.

4. Verfahren zur Bekämpfung von Insekten und Milben, dadurch gekennzeichnet, daß man Verbindungen gemäß Anspruch 1 auf die genannten Schädlinge bzw. deren Lebensraum einwirken läßt.

5. Verwendung von Verbindungen gemäß Anspruch 1 zur Bekämpfung von Insekten und Milben.

6. Verfahren zur Herstellung von insektiziden und akariziden Mitteln, dadurch gekennzeichnet, daß man Verbindungen gemäß Anspruch 1 mit Streckmitteln und/oder oberflächenaktiven Mitteln mischt.